(19) Europäisches Patentamt
European Patent Office
Office européen des brevets

(11) Veröffentlichungsnummer : **0 148 408**
**B1**

# (12) EUROPÄISCHE PATENTSCHRIFT

(45) Veröffentlichungstag der Patentschrift : **02.03.88**

(51) Int. Cl.$^4$ : **C 07 D239/36**

(21) Anmeldenummer : **84114717.6**

(22) Anmeldetag : **05.12.84**

## (54) Verfahren zur Herstellung von 2-Isopropyl-4-methyl-6-hydroxy-pyrimidin.

(30) Priorität : **08.12.83 DE 3344429**

(43) Veröffentlichungstag der Anmeldung : **17.07.85 Patentblatt 85/29**

(45) Bekanntmachung des Hinweises auf die Patenterteilung : **02.03.88 Patentblatt 88/09**

(84) Benannte Vertragsstaaten : **CH DE FR GB IT LI**

(56) Entgegenhaltungen :
**DE-A- 2 065 698**
**DE-A- 2 853 887**
**GB-A- 2 083 814**
**US-A- 4 052 396**
**US-A- 4 052 397**
**US-A- 4 163 848**

(73) Patentinhaber : **WACKER-CHEMIE GMBH**
**Prinzregentenstrasse 22**
**D-8000 München 22 (DE)**

(72) Erfinder : **Deinhammer, Wolfgang, Dr. Dipl.-Ing.**
**Röntgenstrasse 32**
**D-8263 Burghausen (DE)**
Erfinder : **Schilling, Bernd, Dr. Dipl.-Chem.**
**Schopenhauerweg 10**
**D-8263 Burghausen (DE)**

**EP 0 148 408 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Verfahren zur Herstellung von 2-Isopropyl-4-methyl-6-hydroxypyrimidin der Formel

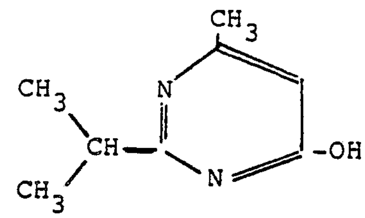

(im folgenden als Hydroxypyrimidin abgekürzt), das als Zwischenprodukt für die Gewinnung von O,O-Diethyl-O-(2-isopropyl-4-methyl-pyrimidyl-6)-thionophosphat benötigt wird, das als schnellwirkendes Kontaktinsektizid unter der geschützten Bezeichnung « Diazinon » im Handel ist, sind bekannt. Neben dem Standardverfahren zur technischen Herstellung, das im wesentlichen über eine aufwendige 4-Stufen-Synthese vorgenommen wird (unter der Bezeichnung « Amidin-Verfahren » bekannt) sollte der Syntheseweg ausgehend von 3-Aminocrotonsäureamid, das aus Diketen und Ammoniak leicht zugänglich ist, aus wirtschaftlichen Gründen vorteilhaft sein.

Verfahren über diesen Syntheseweg sind zwar in der Patentliteratur mehrfach beschrieben, aber bisher offensichtlich noch nicht in technischem Maßstab ausgeführt worden. So wurde beispielsweise 3-Aminocrotonsäureamid mit Isobuttersäureanhydrid in etwa stöchiometrischen Mengen durch Erhitzen unter Rückfluß in Chloroformlösung umgesetzt unter Bildung und Isolierung von 3-Isobutyryl-aminocrotonsäureamid, das anschließend unter basischen Bedingungen zu dem Hydroxypyrimidin cyclisiert werden kann (vgl. JP-AS 6803.363, ref. in Chem. Abst. 69 (1968), Seite 6263).

Die Ausbeuten bei diesem Verfahren sind jedoch sehr mäßig. Sie wurden für die erste Verfahrensstufe mit 62% d. Th. und einschließlich der Cyclisierungsstufe mit 46% d. Th. angegeben, deshalb wurde die Umsetzung von 3-Aminocrotonsäureamid mit Isobuttersäureisobutylester in Gegenwart von Natriumisobutylat durchgeführt, die als Eintopfverfahren ohne Isolierung von Zwischenprodukten direkt zum Hydroxypyrimidin führte (vgl. DE-OS 20 65 698).

Darüberhinaus wurde ein Eintopfverfahren bekannt, das von Diketen und Ammoniak ausgeht und bei dem das als Zwischenprodukt entstehende 3-Aminocrotonsäureamid ebenfalls zusammen mit Isobuttersäurebutylester in Gegenwart von Natriumisobutoxid in Toluol, bzw. in Isobutanollösung bis zu dem als Natriumsalz anfallenden Hydroxypyrimidin cyclisiert und daraus die Verbindung durch Säurezugabe freigesetzt wurde, unter Erzielung von Ausbeuten bis zu 88% (vgl. US-PS 4 052 397).

Da die Cyclisierung des intermediär gebildeten 3-Isobutyrylaminocrotonsäureamids zum Hydroxypyrimidin unter Abspaltung von 1 Mol Wasser erfolgt, das die Zersetzung des Alkoholats begünstigt und die dadurch entstehende Natriumhydroxidlösung die Hydrolyse der Esterkomponente bewirkt, müssen sowohl Alkalialkoholat als auch Ester in beträchtlichem Überschuß eingesetzt werden, was bei der Durchführung derartiger Verfahren im technischen Maßstab sehr unwirtschaftlich ist.

In der Zwischenzeit wurde daher bereits versucht, das 3-Isobutyrylaminocrotonsäureamid durch Acylierung mittels Dimethylketen herzustellen (vgl. DE-OS 28 53 887) und die isolierte Verbindung dann für ein Eintopfverfahren zur Herstellung von Diazinon einzusetzen, bei dem die Cyclisierung mit überschüssiger, wäßriger oder alkoholischer Natriumhydroxidlösung durchgeführt und das als Natriumsalz anfallende Hydroxypyrimidin anschließend in Suspension mit der Phosphorverbindung umgesetzt wurde (vgl. DE-OS 29 07 773).

Die Verwendung von Dimethylketen als Acylierungsmittel ist jedoch nicht nur unwirtschaftlich, da zu seiner Herstellung Isobuttersäureanhydrid benötigt wird, sondern auch nicht ungefährlich, da die Alkylketene bekanntlich zur Bildung explosiver Peroxide neigen. Außerden ist die Isolierung von 3-Isobutyrylaminocrotonsäureamid immer mit Schwierigkeiten verbunden, da diese Verbindung thermolabil und weder im sauren noch im alkalischen Bereich ausreichend stabil ist.

Es stellt sich somit die Aufgabe, ein Verfahren zur Verfügung zu stellen, das die Herstellung des Hydroxypyrimidins, ausgehend von 3-Aminocrotonsäureamid und einem Acylierungsmittel, ohne Isolierung des intermediär entstehenden 3-Isobutyrylaminocrotonsäureamids auf einfachere und wirtschaftlichere Weise ermöglicht, als die bisher bekannten Verfahren.

Diese Aufgabe bei dem Verfahren zur Herstellung von 2-Isopropyl-4-methyl-6-hydroxypyrimidin durch Acylierung von 3-Aminocrotonsäureamid mit einer Isobutyrylverbindung, anschließender Cyclisierung unter alkalischen Bedingungen und Freisetzung der Verbindung mittels einer Säure aus dem als Alkalisalz anfallenden Hydroxypyrimidinderivat wird erfindungsgemäß dadurch gelöst, daß in einer ersten Verfahrensstufe die Acylierung mit überschüssigem Isobuttersäureanhydrid in Gegenwart eines Alkalisalzes der Isobuttersäure bei Temperaturen von 50° bis 120 °C unter Ausschluß eines Lösungsmittels durchgeführt, in einer zweiten Verfahrensstufe die Cyclisierung in dem anfallenden Reaktionsgemisch

durch Zugabe einer wäßrigen Alkalihydroxidlösung bei Temperaturen von 80° bis 105 °C durchgeführt und dann das Hydroxypyrimidin in an sich bekannter Weise isoliert wird.

Für die Acylierung in der ersten Verfahrensstufe können 1,01 bis 1,5 Mol, vorzugsweise 1,05 bis 1,3 Mol Isobuttersäureanhydrid und 0,01 bis 0,4 Mol, vorzugsweise 0,02 bis 0,15 Mol des Alkalisalzes der Isobuttersäure je Mol 3-Aminocrotonsäureamid verwendet werden. Als Alkalisalze der Isobuttersäure sind insbesondere Kalium- und Natriumisobutyrat geeignet, wobei letzteres aufgrund der leichteren Verfügbarkeit bevorzugt ist.

In der zweiten Verfahrensstufe wird eine wäßrige Alkalihydroxid- vorzugsweise Natriumhydroxidlösung verwendet, deren Konzentration im Bereich von 5 bis 60 Gewichtsprozent, vorzugsweise von 10 bis 50 Gewichtsprozent liegen kann.

Außerdem wird in der zweiten Verfahrensstufe die Menge des insgesamt zugefügten Alkalihydroxids vorteilhaft so bemessen, daß sie sowohl für die Neutralisation der bei der Acylierungsreaktion gebildeten und durch Zersetzung von überschüssigem Isobuttersäureanhydrid entstehenden Isobuttersäure, als auch für die Cyclisierung ausreichend ist. Für die Cyclisierungsreaktion selbst werden weniger als 1 Mol Alkalihydroxid je Mol des ursprünglich eingesetzten 3-Aminocrotonsäureamids benötigt, wobei 0,3 bis 0,8 Mol Alkalihydroxid je Mol des ursprünglich, das heißt in der ersten Verfahrensstufe eingesetzten 3-Aminocrotonsäureamids bereits ausreichend sind.

Für die Durchführung des erfindungsgemäßen Verfahrens wird in der ersten Verfahrensstufe vorteilhaft ein Gemisch aus Isobuttersäureanhydrid und Alkaliisobutyrat erhitzt und diesem Gemisch das 3-Aminocrotonsäureamid unter guter Phasendurchmischung und Aufrechterhaltung der Temperatur zugegeben. Die Acylierungsreaktion ist in Abhängigkeit von der gewählten Temperatur nach etwa 0,25 bis 10 Stunden beendet, wobei im allgemeinen bei Temperaturen im Bereich von 60° bis 100 °C etwa 0,5 bis 2 Stunden ausreichend sind.

Nach beendeter Acylierung wird in das anfallende Reaktionsgemisch, das sich bereits auf Reaktionstemperatur befindet, unmittelbar anschließend die wäßrige Alkalihydroxidlösung für die zweite Verfahrensstufe ohne Kühlung und unter kräftiger mechanischer Bewegung eingetragen, wobei die vorhandene Isobuttersäure neutralisiert und die Cyclisierung zu dem Hydroxypyrimidinderivat erreicht wird. Bei Temperaturen von definitionsgemäß 80° bis 105 °C ist die Cyclisierungsreaktion nach etwa 0,5 bis 5 Stunden praktisch vollständig verlaufen.

Das Verfahren kann jedoch auch so durchgeführt werden, daß nach der beendeten Acylierungsreaktion in der ersten Verfahrensstufe ein Teil der gebildeten Isobuttersäure bzw. des überschüssigen Isobuttersäureanhydrids durch Destillation unter vermindertem Druck entfernt wird, sodaß für die anschliessende Cyclisierung und Neutralisation in der zweiten Verfahrensstufe insgesamt geringere Mengen der Alkalihydroxidlösung erforderlich sind. Dadurch können bei Durchführung des Verfahrens im technischen Maßstab erhebliche Flüssigkeitsmengen eingespart werden, was zu einer verbesserten Raum-/Zeitausbeute führt.

Die weitere Aufarbeitung zur Gewinnung des freien Hydroxypyrimidins kann in an sich bekannter Weise vorgenommen werden. Hierzu wird das abgekühlte Reaktionsgemisch mit einer Mineralsäure, wie wäßriger Salzsäure, neutralisiert. Der ausgefallene Feststoff kann durch Zentrifugieren abgetrennt und mit Wasser gewaschen werden. Die Mutterlauge enthält neben dem Alkaliisobutyrat noch etwas Hydroxypyrimidin, das durch Extraktion mit organischen Lösungsmitteln, die mit Wasser nicht mischbar sind, wie Methylenchlorid, Chloroform, 1,2-Dichlorethan, Benzol, Toluol oder Xylol gewonnen werden kann. Es ist jedoch auch möglich, die gesamte Suspension, die nach der Säurezugabe anfällt, mit einem der oben genannten Lösungsmittel zu extrahieren und nach dem Eindampfen der Extrakte das Hydroxypyrimidin zu isolieren. Das so erhaltene Hydroxypyrimidin ist im allgemeinen genügend rein und kann gegebenenfalls durch Umkristallisation noch weiter gereinigt werden.

Aus dem nach der Abtrennung des Hydroxypyrimidins in der wäßrigen Phase vorhandenen Alkaliisobutyrat kann durch weitere Säurezugabe die Isobuttersäure freigesetzt und diese durch Schichtentrennung isoliert werden.

Nach dem erfindungsgemäßen Verfahren werden Ausbeuten an 2-Isopropyl-4-methyl-6-hydroxypyrimidin von mindestens 90% d.Th., bezogen auf eingesetztes 3-Aminocrotonsäureamid erzielt.

## Beispiel

In einem Reaktionsgefäß von 1 l Fassungsvermögen werden 173,8 g (1,1 Mol) Isobuttersäureanhydrid und 10 g (0,09 Mol) Natriumisobutyrat vorgelegt und auf 80 °C erhitzt. In dieses Gemisch werden unter Rühren und gelegentlichem Kühlen 100 g (1,0 Mol) 3-Aminocrotonsäureamid so langsam eingetragen, daß die Temperatur 85 °C nicht übersteigt. Nach beendeter Zugabe wird das Rühren 30 Minuten unter Aufrechterhaltung der Temperatur fortgesetzt.

Anschließend werden 230 ml einer 25 Gew.-%-igen Natriumhydroxidlösung zugegeben (das entspricht 1,84 Mol NaOH insgesamt ; abzüglich der für die Neutralisation der vorhandenen Isobuttersäure verbrauchten Menge, stehen für die Cyclisierung 0,64 Mol NaOH je Mol des eingesetzten 3-Aminocrotonsäureamids zur Verfügung) und das Reaktionsgemisch 1 Stunde unter Rückfluß erhitzt.

Dann wird das Reaktionsgemisch abgekühlt und mit 20 Gew.-%-iger Salzsäure neutralisiert. Der ausgefallene Feststoff wird abgesaugt, mit wenig Wasser gewaschen und getrocknet. Es werden 116,2 g

2-Isopropyl-4-methyl-6-hydroxypyrimidin erhalten.

Die Mutter- und Waschlaugen werden mehrmals mit Methylenchlorid ausgeschüttelt. Der Extrakt wird einmal mit Wasser gegengeschüttelt. Nach dem Abdestillieren des Lösungsmittels verbleiben 20,8 g 2-Isopropyl-4-methyl-6-hydroxypyrimidin, das entspricht einer Gesamtausbeute von 90,1% d. Th., bezogen auf eingesetztes 3-Aminocrotonsäureamid.

## Patentansprüche

1. Verfahren zur Herstellung von 2-Isopropyl-4-methyl-6-hydroxypyrimidin durch Acylierung von 3-Aminocrotonsäureamid mit einer Isobutyrylverbindung, anschließende Cyclisierung unter alkalischen Bedingungen und Freisetzung der Verbindung mittels einer Säure aus dem als Alkalisalz anfallenden Hydroxypyrimidinderivat, dadurch gekennzeichnet, daß in einer ersten Verfahrensstufe die Acylierung mit überschüssigem Isobuttersäureanhydrid in Gegenwart eines Alkalisalzes der Isobuttersäure bei Temperaturen von 50° bis 120 °C unter Ausschluß eines Lösungsmittels durchgeführt, wobei je Mol 3-Aminocrotonsäureamid 1,01 bis 1,5 Mol Isobuttersäureanhydrid und 0,01 bis 0,4 Mol des Alkalisalzes der Isobuttersäure verwendet werden, in einer zweiten Verfahrensstufe die Cyclisierung in dem anfallenden Reaktionsgemisch durch Zugabe einer wäßrigen Alkalihydroxidlösung bei Temperaturen von 80° bis 105 °C durchgeführt und dann das Hydroxypyrimidin in an sich bekannter Weise isoliert wird.

2. Verfahren nach Anspruch 1, dadurch gekennzeichnet, daß in der zweiten Verfahrensstufe eine 10 bis 50 Gew.-%-ige wäßrige Alkalihydroxidlösung verwendet wird.

3. Verfahren nach Anspruch 1, dadurch gekennzeichnet, daß in der zweiten Verfahrensstufe die Menge des insgesamt zugefügten Alkalihydroxids so bemessen wird, daß sie sowohl für die Neutralisation der bei der Acylierung gebildeten und durch Zersetzung von überschüssigem Isobuttersäureanhydrid entstehenden Isobuttersäure, als auch für die Cyclisierung ausreichend ist.

4. Verfahren nach Anspruch 3, dadurch gekennzeichnet, daß für die Cyclisierung in der zweiten Verfahrensstufe 0,3 bis 0,8 Mol Alkalihydroxid je Mol des in der ersten Verfahrensstufe eingesetzten 3-Aminocrotonsäureamids verwendet werden.

## Claims

1. Process for preparing 2-isopropyl-4-methyl-6-hydroxypyrimidine by acylation of 3-aminocrotonamide with an isobutyryl compound, subsequent cyclization under alkaline conditions and liberation of the compound by means of an acid from the hydroxypyrimidine derivative obtained in the form of an alkali metal salt, characterized in that in a first process stage the acylation is carried out with excess isobutyric anhydride in the presence of an alkali metal salt of isobutyric acid at temperatures of 50° to 120 °C in the absence of a solvent, 1.01 to 1.5 moles of isobutyric acid anhydride and 0.01 to 0.4 mole of the alkali metal salt of isobutyric acid being used per mole of 3-aminocrotonamide, in a second process stage the cyclization is carried out in the resulting reaction mixture by addition of an aqueous alkali metal hydroxide solution at temperatures of 80° to 105 °C, and the hydroxypyrimidine is then isolated in a conventional manner.

2. Process according to Claim 1, characterized in that in the second process stage a 10 to 50% strength by weight aqueous alkali metal hydroxide solution is used.

3. Process according to Claim 1, characterized in that in the second process stage the amount of alkali metal hydroxide added in total is dimensioned in such a way that it is sufficient not only for neutralizing the isobutyric acid formed in the course of the acylation and produced by decomposition of excess isobutyric anhydride but also for the cyclization.

4. Process according to Claim 3, characterized in that 0.3 to 0.8 mole of alkali metal hydroxide per mole of the 3-aminocrotonamide used in the first process stage are used for the cyclization in the second process stage.

## Revendications

1. Procédé pour préparer l'isopropyl-2 méthyl-4 hydroxy-6 pyrimidine par acylation de l'amino-3 crotonamide au moyen d'un composé isobutyrylique, puis cyclisation dans des conditions alcalines et libération du composé au moyen d'un acide à partir du dérivé d'hydroxypyrimidine obtenu à l'état de sel de métal alcalin, procédé caractérisé en ce que, dans une première étape, on effectue l'acylation avec un excès d'anhydride isobutyrique, en présence d'un sel de métal alcalin de l'acide isobutyrique, à des températures de 50 à 120 °C et sans solvant, en mettant en jeu, par mole d'amino-3 crotonamide, de 1,01 à 1,5 mol d'anhydride isobutyrique et de 0,01 à 0,4 mol du sel de métal alcalin de l'acide isobutyrique, puis, dans une seconde étape, on effectue la cyclisation, dans le mélange réactionnel formé, par addition d'une solution aqueuse d'un hydroxyde de métal alcalin, à des températures de 80° à 105 °C, et enfin on isole l'hydroxypyrimidine de manière connue.

2. Procédé selon la revendication 1 caractérisé en ce que, dans la seconde étape, on utilise une solution aqueuse d'un hydroxyde de métal alcalin d'une concentration comprise entre 10 et 50% en poids.

3. Procédé selon la revendication 1 caractérisé en ce que, dans la deuxième étape, on règle la quantité de l'ensemble de l'hydroxyde de métal alcalin que l'on a ajouté de telle façon qu'il y en ait suffisamment non seulement pour la neutralisation de l'acide isobutyrique formé lors de l'acylation et de l'acide isobutyrique formé par décomposition de l'excès d'anhydride isobutyrique, mais encore pour la cyclisation.

4. Procédé selon la revendication 3 caractérisé en ce qu'on utilise, pour la cyclisation dans la seconde étape, de 0,3 à 0,8 mole d'hydroxyde de métal alcalin par mole de l'amino-3 crotonamide mis en jeu dans la première étape.